(19) Europäisches Patentamt
European Patent Office
Office européen des brevets

(11) Numéro de publication: **0 465 936 B1**

(12) **FASCICULE DE BREVET EUROPEEN**

(45) Date de publication de fascicule du brevet: **25.01.95**

(51) Int. Cl.[6]: **C07C 49/447**, C07C 49/637, C07C 45/51, C07C 45/67, C07C 45/62, A61K 7/46, C11B 9/00

(21) Numéro de dépôt: **91110627.6**

(22) Date de dépôt: **27.06.91**

(54) **Cétones décaliniques, leur utilisation en parfumerie et procédé pour leur préparation.**

(30) Priorité: **09.07.90 CH 2283/90**

(43) Date de publication de la demande:
**15.01.92 Bulletin 92/03**

(45) Mention de la délivrance du brevet:
**25.01.95 Bulletin 95/04**

(84) Etats contractants désignés:
**CH DE FR GB LI NL**

(56) Documents cités:
**US-A- 3 932 516**

**TETRAHEDRON LETTERS. vol. 31, no. 28, 6 Juillet 1990, OXFORD GB pages 4021 -4024; C. FEHR ET AL.: 'Diastereocontrolled Synthesis of Functionalized Trans-Decalins via Electrocyclic Reaction of Trienol Ethers.'**

(73) Titulaire: **FIRMENICH SA**
**1, route des Jeunes**
**CH-1211 Genève 8 (CH)**

(72) Inventeur: **Fehr, Charles**
**6, Chemin Ravoux**
**CH-1290 Versoix (CH)**
Inventeur: **Galindo, José**
**7, chemin Croix-du-Levant**
**CH-1220 Les Avanchets (CH)**
Inventeur: **Guntern, Olivier**
**16, route de Malagnou**
**CH-1208 Genève (CH)**

(74) Mandataire: **Salvadori, Giuseppe, Dr.**
**c/o Firmenich S.A.**
**Case Postale 239**
**CH-1211 Genève 8 (CH)**



Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a trait au domaine de la parfumerie. Elle concerne plus particulièrement des cétones décaliniques de formule

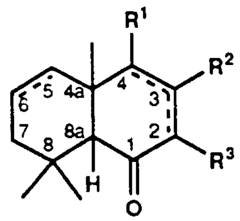

(I)

pouvant avoir une double liaison dans l'une des positions indiquées par les lignes pointillées ou deux doubles liaisons en positions 2 et 5 ou 3 et 5, telles qu'indiquées par les lignes pointillées, et dans laquelle les symboles $R^1$, $R^2$ et $R^3$ représentent un atome d'hydrogène ou un radical méthyle, à l'exclusion de la perhydro-4a,8,8-triméthyl-1-trans-naphtalénone.

Nombreux sont les composés odorants de structure décalinique que l'on connaît à ce jour. En particulier, une variété de cétones décaliniques de structure plus ou moins proche de celle des composés (I) sont décrites dans l'art antérieur. G. Ohloff, par exemple, dans un article de revue, résume les propriétés odorantes de bon nombre de ces cétones connues, et analyse ces propriétés dans le contexte d'une étude plus générale ayant trait aux rapports structure-activité pour les composés possédant des notes olfactives du type d'ambregris [voir Fragrance Chemistry : The Sense of Smell, page 535, ed. E.T. Theimer, AP, USA (1982), et références y citées].

La plupart des cétones décaliniques connues de l'art antérieur dont la structure est analogue à, ou homologue de celle des composés (I) selon l'invention possèdent le groupe carbonyle dans une position du cycle moins encombrée et/ou plus éloignée du groupe diméthyle que dans la formule (I). Ceci résulte du fait que l'on ne disposait pas, jusqu'à maintenant, de procédés permettant de préparer, à l'état pur et à l'échelle industrielle, des cétones décaliniques de formule (I).

Une seule hydronaphtalénone obéissant à la formule (I) est connue à ce jour, à savoir la perhydro-4a,8,8-triméthyl-1-trans-naphtalénone de formule

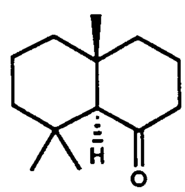

(A)

qui est mentionnée par G. Ohloff [ref. citée]. Cependant, ce composé n'a pas, de toute évidence, été obtenu à l'état pur, car l'auteur susmentionné affirme que l'odeur qui lui est attribuée, à savoir une forte odeur résineuse, de type vernis, accompagnée d'une note sous-jacente de moisi, est en partie due à la contribution olfactive d'une cétone analogue de formule

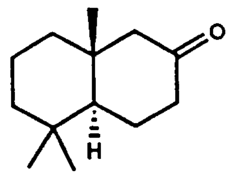

D'autre part, A.K. Banerjee et al. [voir Tetrahedron 37, 2749 (1981) et J. Chem. Soc. Perkin Trans. I 1982, 2547] ont réussi à préparer la cétone (A) et décrivent son utilisation dans la synthèse des cétones décaliniques suivantes :

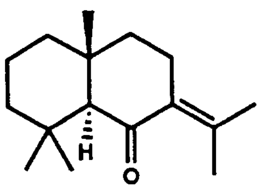

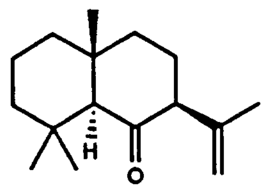

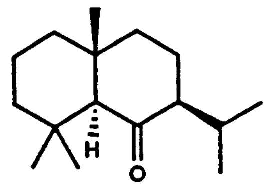

Selon ces auteurs, ces dernières ne peuvent être obtenues avec un bon rendement que par un procédé complexe à plusieurs étapes, ne présentant qu'un intérêt académique. Par ailleurs, la cétone de départ de formule (A) est préparée par un procédé d'au moins quatre étapes, parmi lesquelles on retrouve une réaction d'hydroboration-oxydation d'une octaline qui, pour des raisons de sécurité, ne se prête pas à une application à l'échelle industrielle. De même la préparation de ladite octaline entraîne des réactions peu appropriées à une synthèse industrielle de ce composé.

Finalement, le procédé de préparation de la cétone (A) décrit dans l'art antérieur cité ci-dessus ne permet pas l'obtention de cétones insaturées de formule (I) ou, du moins, exige le concours d'étapes préparatives supplémentaires qui rendraient la synthèse de ces composés encore moins praticable à l'échelle industrielle.

Il convient de noter que Banerjee et al. ont décrit uniquement la synthèse des composés susmentionnés et n'ont suggéré aucune application intéressante, du point de vue olfactif, de ces composés.

Nous avons maintenant découvert que non seulement la cétone (A) connue de l'art antérieur, mais encore d'autres cétones décaliniques, saturées ou insaturées, représentées par la formule (I) pouvaient, grâce à un procédé original qui fait également l'objet de la présente invention, être obtenues avec un bon rendement, de façon simple, faisant usage de réactions pouvant être utilisées à l'échelle industrielle d'une façon sûre et efficace.

Par ailleurs, c'est avec surprise que nous avons découvert que les cétones décaliniques de formule (I) définie auparavant possédaient des propriétés odorantes très intéressantes et distinctes de celles des cétones décaliniques connues de l'art antérieur. En effet, bien que conservant le caractère boisé-ambré qui semble être typique des composés de ce type [voir G. Ohloff, référence citée], les cétones (I) de l'invention exhalent des odeurs tout à fait surprenantes et uniques en parfumerie, dans lesquelles cette note boisée-ambrée peut être accompagnée d'une note florale qui rappelle celle des damascones. Cette combinaison de caractères odorants est inconnue dans ce type de composés. En plus, suivant la nature spécifique du composé (I), son odeur peut être encore distinguée par d'autres nuances olfactives, ce qui rend l'utilisation de ces composés en parfumerie particulièrement variée.

Nous avons également constaté que, pour une même structure de formule (I), le caractère odorant était différent d'une forme isomérique à l'autre. En effet, et si l'on ne tient pas compte de l'orientation particulière des substituants $R^1$, $R^2$ ou $R^3$ , les composés de l'invention peuvent se présenter sous deux formes isomériques, résultant de la configuration du radical méthyle en position 4a du cycle naphtalénique, qui peut être cis ou trans par rapport à l'atome d'hydrogène en position 8a. Il a été observé que les isomères de configuration trans, représentés par la formule

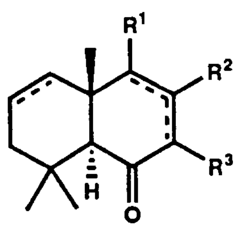

(Ia)

dans laquelle les lignes pointillées et les symboles $R^1$, $R^2$ et $R^3$ ont le sens indiqué à la formule (I), possédaient les notes odorantes les plus intéressantes et étaient, de ce fait, des composés préférés selon l'invention.

Par exemple, la 4a,5,6,7,8,8a-hexahydro-4a,8,8-triméthyl-1(4H)-trans-naphtalénone, un composé préféré de l'invention, possède une note boisée, damasconée, avec un élégant côté camphré. Sa note de fond évoque le para-tert-butylcyclohexanol, avec un caractère ressemblant à celui de la géosmine [voir US 4'248'742] et aussi ambré-terreux.

D'autre part, la 4a,5,6,7,8,8a-hexahydro-3,4a,8,8-tétraméthyl-1(4H)-trans-naphtalénone, que l'on cite également à titre préférentiel, exhale une odeur ambrée et boisée moins puissante que celle de son homologue précité, mais dotée d'un caractére poudré-iononé et damasconé plus net.

Un troisième composé préféré selon l'invention est la perhydro-4a,8,8-triméthyl-1-trans-naphtalénone qui possède le même caractère terreux et para-tert-butylcyclohexanol du composé cité en premier lieu, mais dont l'odeur est plus ambrée, légèrement fleurie-musquée et camphrée que celle de ce dernier et possède un très intéressant caractère patchouli.

Cette variété de nuances olfactives peut encore être constatée dans les autres composés (I) selon l'invention, dont les propriétés odorantes sont décrites en détail dans les exemples de préparation présentés plus loin.

Grâce à leurs propriétés odorantes, les composés de formule (I) ou (Ia) selon l'invention peuvent être utilisés avantageusement, aussi bien en parfumerie fine que dans des applications fonctionnelles, pour la préparation de compositions parfumantes et articles parfumés. Parmi ces derniers on peut citer les parfums ou eaux de toilette, les savons, les gels de douche ou bain, les shampoings et autres produits d'hygiène capillaire, les préparations cosmétiques et les déodorants corporels. Ils trouvent également un emploi utile dans le parfumage de détergents, d'adoucissants textiles ou de produits d'entretien.

Les concentrations dans lesquelles les composés de l'invention peuvent être employés pour obtenir les effets odorants désirés varient dans une gamme de valeurs très étendue. On sait que ces concentrations sont fonction des conditions d'utilisation, c'est-à-dire, elles varient suivant que le composé est utilisé seul ou en mélange avec d'autres ingrédients, solvants ou adjuvants usuels en parfumerie, ainsi que de la nature de l'effet parfumant recherché. Lorsque, comme il est courant, les composés (I) sont utilisés en mélange avec d'autres ingrédients, leur concentration dépendra aussi de la nature des co-ingrédients parfumants.

A titre d'exemple, on peut citer des concentrations de l'ordre de 1 à 5%, voire 10 ou même 20%, en poids de composé (I), par rapport au poids de la composition dans laquelle ce composé est incorporé. Ces valeurs peuvent être nettement inférieures lorsque le composé (I) est utilisé pour le parfumage des articles de consommation divers susmentionnés.

Comme il a été cité auparavant, la présente invention a également pour objet un procédé pour la préparation des cétones décaliniques de formule

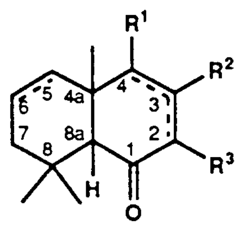

(I)

pouvant avoir une double liaison dans l'une des positions indiquées par les lignes pointillées ou deux doubles liaisons en positions 2 et 5 ou 3 et 5, telles qu'indiquées par les lignes pointillées, et dans laquelle les symboles $R^1$, $R^2$ et $R^3$ représentent un atome d'hydrogène ou un radical méthyle, caractérisé en ce qu'il comprend les étapes suivantes :

a) le traitement thermique d'un composé de formule

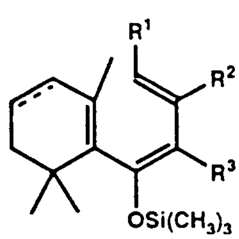

(II)

dans laquelle la ligne pointillée indique l'emplacement d'une liaison simple ou double et les symboles $R^1$, $R^2$ et $R^3$ sont définis comme à la formule (I), suivie d'un traitement acide du produit de réaction ainsi obtenu et de la séparation d'un composé de formule

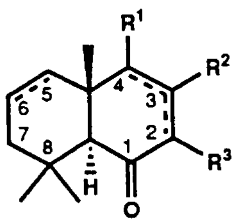

(Ia)

dans laquelle les symboles $R^1$, $R^2$ et $R^3$ ont le sens indiqué à la formule (I) et les lignes pointillées indiquent l'emplacement d'une liaison simple ou double en position 5 et d'une liaison double en position 2 ou 3 du cycle naphtalénique;

b) le cas échéant, l'épimérisation, suivant des procédés connus en soi, dudit composé de formule (Ia) définie en a) pour obtenir son isomère de configuration cis de formule

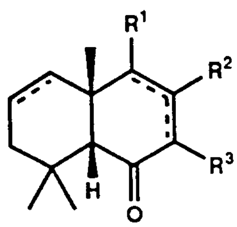

(Ib)

dans laquelle les lignes pointillées et les symboles $R^1$, $R^2$ et $R^3$ ont le sens indiqué à la formule (Ia);

et

c) le cas échéant, la réduction sélective, de façon connue en soi, dudit composé de formule (Ia), respectivement de formule (Ib), pour obtenir un composé de formule

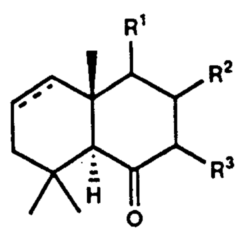

(I'a)

respectivement de formule

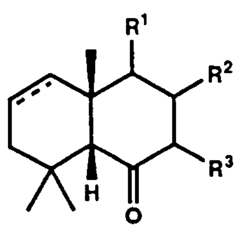

(I'b)

dans laquelle la ligne pointillée indique l'emplacement d'une liaison simple ou double et les symboles $R^1$, $R^2$ et $R^3$ ont le sens indiqué ci-dessus.

L'étape principale du procédé de l'invention consiste en une réaction de clôture électrocyclique, obtenue par traitement thermique du composé de formule (II) dans un solvant organique inerte. Ce traitement thermique peut être une réaction de pyrolyse effectuée conformément à des techniques courantes. Typiquement, une solution du produit à pyrolyser dans un solvant inerte est injectée à une des

extrémités d'une colonne de quartz préalablement chauffée à une température prédéterminée. L'opération peut être effectuée sous azote ou pression réduite, et les vapeurs du pyrolysat sont condensées dans une trappe refroidie à une température très basse à l'aide d'un mélange refroidissant approprié. La température de pyrolyse peut varier dans une gamme de valeurs relativement étendue, par exemple, entre 300 et 400 °C. De préférence, la température de la réaction ne doit pas dépasser les 400 °C, pour éviter la formation de produits secondaires indésirables. Selon un mode d'exécution préférentiel du procédé de l'invention, cette température se situe autour de 365 °C. Cependant, cette température est fonction de la longueur de la colonne de quartz et dépend notamment de la valeur de la pression appliquée. L'homme de l'art est à même d'adapter ces paramètres, et d'autres tel que le temps de pyrolyse, de façon à obtenir un bon rendement en produit final désiré.

Le pyrolysat condensé, qui contient des éthers silylés, est ensuite traité en milieu acide pour enlever les groupes labiles, et le mélange ainsi traité est purifié à l'aide des techniques usuelles telles que distillation fractionnée, chromatographie et cristallisation, pour fournir le composé de formule (Ia) définie ci-dessus.

Alternativement, le traitement thermique du composé (II) peut être effectué à une température inférieure, en autoclave et en solution dans un solvant organique inerte. Les conditions spécifiques de ce type de méthode sont décrites en détail dans les exemples présentés plus loin.

Les produits de départ de formule (II) peuvent être obtenus à partir d'esters ou de cétones disponibles sur le marché, selon deux procédés alternatifs représentés dans le schéma suivant, en utilisant des méthodes analogues à celles décrites par C. Fehr et ai. dans Helv. Chim. Acta 69, 228 (1986) et 70, 1745 (1987) :

SCHEMA I

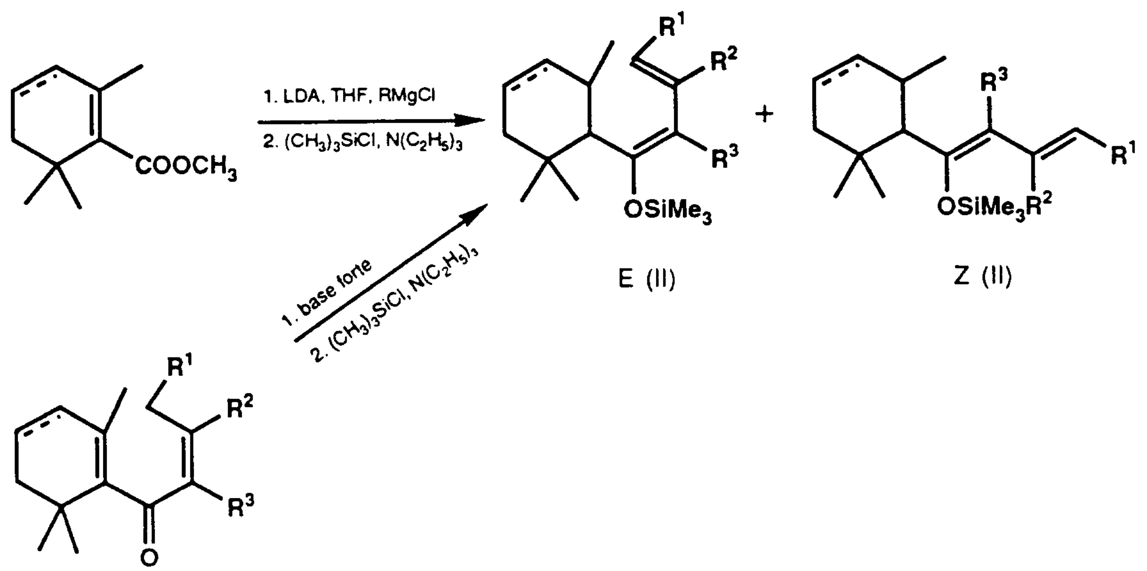


E (II) Z (II)

RMgCl = 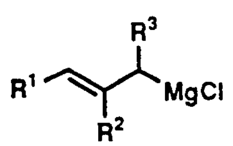

Me = $CH_3$
LDA = lithiumdiisopropylamine
THF = tétrahydrofuranne

La première méthode, à partir de l'ester représenté, consiste en une réaction de Grignard en milieu basique, suivie de silylation de l'énolate résultant. Le produit de la réaction est un mélange riche en

l'isomère E(II) désiré, dont la cyclisation, suivie d'un traitement acide pour enlever le groupe labile, permet d'obtenir une cétone décalinique (Ia) de configuration trans, comme cité auparavant.

Le deuxième procédé comprend la déprotonation, à l'aide d'une base forte, de la cétone illustrée, suivie de silylation de l'énolate obtenu. La diastéréosélectivité de la réaction peut être contrôlée avec haute précision par un choix avisé de la base déprotonante, comme il ressort des exemples de préparation présentés plus loin, où les conditions spécifiques des réactions décrites ci-dessus sont exposées de façon détaillée.

Selon le procédé de l'invention, les cétones décaliniques de formule (Ia), dans laquelle les symboles $R^1$, $R^2$ et $R^3$ ont le sens indiqué pour la formule (I) et les lignes pointillées indiquent l'emplacement d'une liaison simple ou double en position 5 et d'une liaison double en position 2 ou 3 du cycle naphtalénique, obtenues dans les réactions précédentes, peuvent être transformées en leurs isomères de configuration cis de formule (Ib) à l'aide de réactions d'épimérisation de type classique.

D'autre part, les cétones insaturées précitées de configuration cis ou trans peuvent être soumises à des réactions de réduction sélective pour fournir les cétones de configuration cis ou trans de formule (I'a) et (I'b) définies auparavant. Il s'agit de réactions de type courant telles que des hydrogénations ou des réductions à l'aide d'agents réducteurs courants, comme par exemple des métaux alcalins, l'aluminohydrure de lithium ou des hydrures de trialkylétain [voir par exemple H.G. Kuivila et al., J. Am. Chem. Soc. 83, 1246 (1961) et H.R. Wolf et al., Helv. Chim. Acta 56, 1062 (1973)].

Les conditions spécifiques de ces réactions sont décrites en détail dans les exemples de préparation présentés plus loin.

L'invention sera maintenant décrite plus en détail à l'aide des exemples de préparation présentés ci-après, dans lesquels les températures sont indiquées en degrés centigrades et les abréviations ont le sens usuel dans l'art.

L'invention sera également illustrée à l'aide d'exemples d'application en parfumerie.

Exemple 1

Préparation de 4a,5,6,7,8,8a-hexahydro-4a,8,8-triméthyl-1(4H)-trans-naphtalénone

82,0 g de 1,3,3-triméthyl-2-[1-(triméthylsilyloxy)-1,3-butadiényl]-1-cyclohexène (mélange E/Z = 11:1 ; chromatographiquement pur à 95% ; 295 mmole) dans le cyclohexane (400 ml) ont été conduits à une vitesse de 0,8 ml/min à travers une colonne de quartz de 4 m préalablement chauffée à 365° et maintenue sous un courant constant d'azote (0,8 ml/min). Le pyrolysat (78,0 g) a été concentré dans un récipient équipé d'un réfrigérant à la neige carbonique. Le produit résultant consistant en un mélange contenant 1,2,3,4,4a,5-hexahydro-1,1,4a-triméthyl-8-(triméthylsilyloxy)-naphtalène, 1,2,3,4,4a,8a-hexahydro-1,1,4a-triméthyl-8-(triméthylsilyloxy)-trans-naphtalène, 1,2,3,4,5,6-hexahydro-1,1,4a,6-tétraméthyl-8-(triméthylsilyloxy)-naphtalène et produit de départ, a été dissous dans le THF (400 ml), traité avec HCl à 5% (10 ml) et agité pendant 1 h à 20°. Après distillation fractionnée (55°/6,7 Pa) et cristallisation (éther de pétrole/-78°) on a obtenu 18,2 g de la naphtalénone désirée (rend. 32%). Les eaux-mères ont été purifiées par chromatographie ($SiO_2$, cyclohexane/acétate d'éthyle = 98:2) et cristallisées pour fournir 6,0 g (rend. 11%) de la même naphtalénone. D'autres fractions distillées moins pures et les eaux-mères restantes contenaient ~ 16 g (rend. 29%) du produit désiré.

| | |
|---|---|
| IR : | 2920, 1675, 1460, 1380, 1225, 1125 $cm^{-1}$. |
| RMN($^1$H, 360MHz) : | 1,04(s, 3H) ; 1,14(s, 3H) ; 1,16(s, 3H) ; 1,17-1,25(m, 1H) ; 1,35-1,49(m, 3H) ; 1,53-1,71(m, 2H) ; 2,09(dd, J = 18, 6,5Hz, 1H) ; 2,19(s, 1H) ; 2,33(large d, J = 18Hz, 1H) ; 5,89(dd, J = 10, 3,5Hz, 1H) ; 6,68(ddd, J = 10, 6,5, 2,5Hz, 1H) $\delta$ ppm. |
| RMN($^{13}$C) : | 18,2(t) ; 20,4(q) ; 21,3(q) ; 32,1(s) ; 33,3(q) ; 39,1(s) ; 41,7(t) ; 43,6(t) ; 46,4(t) ; 62,5(d) ; 130,9(d) ; 144,5(d) ; 200,7(s) $\delta$ ppm. |
| SM : | 192(20, $M^+$), 177(7), 149(8), 121(16), 109(100), 81(11), 68(13), 55(16), 41(42). |

Note odorante : décrite plus haut.

Le 1,3,3-triméthyl-2-[1-(triméthylsilyloxy)-1,3-butadiényl]-1-cyclohexène de départ à été préparé ainsi :

a) à partir de $\beta$-cyclogéraniate de méthyle

Une solution de butyllithium dans l'hexane (118 ml, 1,40N, 164,8 mmole) a été ajoutée, à une température de -10 à 0°, à une solution agitée de diisopropylamine (17,20 g, 24,3 ml, 170,0 mmole) dans le THF (120 ml). Une fois l'addition terminée, la solution jaune a été traitée avec une solution de chlorure d'allylmagnésium dans le THF (119,0 ml, 1,38N, 164,6 mmole) à 20°, et on a ajouté à la solution bleu-gris du $\beta$-cyclogéraniate de méthyle (20,0 g, 109,8 mmole) à 35-40°. Le mélange jaune

résultant a été agité à cette température pendant 1 h, refroidi à -30° et traité avec $(C_2H_5)_3N$ (22,2 g, 30,4 ml, 219,6 mmole) et $(CH_3)_3SiCl$ (35,6 g, 41,4 ml, 329,4 mmole). On a enlevé le bain de refroidissement et continué l'agitation pendant 2 h à 20°. Le mélange jaune à été traité avec une solution saturée de $NaHCO_3$/glace/éther de pétrole, et extrait.

La phase organique a été lavée à l'eau et NaCl saturé, séchée sur $Na_2SO_4$, évaporée et distillée (55-60°/6,7 Pa). On a obtenu 24,4 g de 1,3,3-triméthyl-2-[1-(triméthylsilyloxy)-1,3-butadiényl]-1-cyclohexène sous forme d'un mélange d'isomères E/Z = 3:1 (95% pur, rend. 80%).

IR : 2920, 1620, 1580, 1455, 1245, 1205, 1160, 1060 $cm^{-1}$.

Isomère E

RMN($^1$H, 360MHz) : 0,24(s, 9H) ; 0,96(s, 3H) ; 1,11(s, 3H) ; 1,44(m, 2H) ; 1,59(s, 3H) ; 1,60-1,74-(m, 2H) ; 1,92-2,07(m, 2H) ; 4,72(dd, J = 10, 2Hz, 1H); 4,93(dd, J = 17, 2Hz, 1H) ; 5,49(d, J = 10Hz, 1H) ; 6,05(ddd, J = 17, 10, 10Hz, 1H) δ ppm.

Pos. NOE entre 0,24(↝) et 5,49 δ ppm [voir T.H. Keller et al., J. Org. Chem. 52, 1870(1987)].

RMN($^{13}$C, 360MHz) : 0,5(q) ; 19,0(t) ; 21,4(q) ; 28,7(q) ; 29,6(q) ; 31,7(t) ; 33,9(s) ; 39,6(t) ; 110,7(t) ; 112,1(d) ; 132,0(s) ; 135,1(d) ; 154,1(s) δ ppm.

SM : 264(3, $M^+$), 249(6), 194(9), 179(34), 105(12), 91(14), 75(35), 73(100), 55(10), 45(15).

Isomère Z

RMN($^1$H, 360MHz) : signaux significatifs : 0,17(s, 9H) ; 1,70(s, 3H) ; 4,84(dd, J = 10, 2Hz, 1H) ; 4,99(dd, J = 17, 2Hz, 1H) ; 5,16(d, J = 10Hz, 1H) ; 6,63(ddd, J = 17, 10, 10Hz, 1H) δ ppm.

RMN($^{13}$C, 360MHz) : signaux détectés : 0,9(q) ; 22,2(q) ; 29,4(q) ; 31,9(t) ; 39,8(t) ; 111,7(t) ; 115,3-(d) ; 132,0(d) δ ppm.

SM : 264(3, $M^+$), 249(6), 194(10), 179(38), 105(12), 91(15), 75(35), 73(100), 55(11), 45(15).

b) à partir de β-damascone

Une solution de β-damascone (5,0 g, 26,0 mmole) dans le THF (20 ml) a été ajoutée goutte à goutte à une solution refroidie (-75°) de $NaN[Si(CH_3)_3]_2$ (Aldrich, 1,0 M dans le THF, 30 ml, 30,0 mmole) dans le THF (30 ml). Après 15 min, la déprotonation était complète. On a ajouté à la même température, du $(CH_3)_3SiCl$ (3,40 g, 3,95 ml, 31,4 mmole) pendant 2 min. Après 1 h, la solution claire de couleur jaune a été traitée avec $N(C_2H_5)_3$ (2,62 g, 3,60 ml, 26,0 mmole), $NaHCO_3$ aqueux saturé/glace/éther de pétrole et extraite pour fournir, après distillation au four à boules (temp. bain 80°/8 Pa), 6,01 g de 1,3,3-triméthyl-2-[1-(triméthylsilyloxy)-1,3-butadiényl]-1-cyclohexène sous forme d'un mélange d'isomères E/Z = 11:1 (rend. 88%).

En répétant ce procédé avec $LiN[Si(CH_3)_3]_2$, respectivement $KN[Si(CH_3)_3]_2$, à la place de $NaN[Si(CH_3)_3]_2$, on a obtenu des mélanges d'isomères E/Z = 7:1 (rend. 93%), respectivement E/Z = 8:1, avec des temps de déprotonation d'environ 20 h, respectivement 1 h.

Les produits ainsi obtenus présentaient les mêmes données analytiques que le mélange obtenu en a).

## Exemple 2

### Préparation de 4a,5,6,7,8,8a-hexahydro-4a,8,8-triméthyl-1(4H)-cis-naphtalénone

Ce composé a été obtenu par épimérisation de son isomère trans préparé dans l'exemple 1. La trans-décaline (500 mg, 2,6 mmole) a été chauffée à reflux dans le toluène (25 ml) en présence d'acide p-toluènesulfonique hydraté (p-TsOH.$H_2O$, 495 mg, 2,6 mmole) pendant 40 h. Le mélange de la réaction, une fois refroidi, a été versé sur $H_2O$ et extrait à l'éther. La phase organique a été lavée 2 fois avec $NaHCO_3$ et une fois à la saumure, ensuite séchée et évaporée (460 mg). On a purifié le produit obtenu sur colonne de silice, en utilisant comme éluant un mélange hexane/chlorure de méthylène 1:1 et, ensuite, chlorure de méthylène. Après distillation on a obtenu 332 mg du produit désiré (rend. 66%).

P. f. 70-78,5°.

IR : 2900, 1640, 1450, 1370, 1240, 1130 $cm^{-1}$.

RMN($^1$H, 360MHz) : 0,88(s, 3H) ; 0,97(s, 3H) ; 0,98(s, 3H) ; 1,14-1,26(m, 2H) ; 1,39-1,71(m, 4H) ; 1,82(s, 1H) ; 1,84(dd, J = 20, 6Hz, 1H) ; 2,71(large d, J = 20Hz, 1H) ; 6,01(dd, J = 10, 2,5Hz, 1H) ; 6,82(m, 1H) δ ppm.

RMN($^{13}$C) : 18,7(t) ; 22,2(q) ; 31,6(q) ; 32,8(q) ; 34,0(s) ; 35,2(s) ; 35,6(t) ; 39,4(t) ; 41,5(t) ; 63,3(s) ; 130,1(d) ; 147,7(d) ; 202,2(s) δ ppm.

SM : 192(6, $M^+$), 177(2), 149(4), 121(7), 109(100), 97(4), 81(6), 79(6), 67(5), 55(4), 41-(6).

Exemple 3

Préparation de 4a,5,6,7,8,8a-hexahydro-3,4a,8,8-tétraméthyl-1(4H)-trans-naphtalénone

55,5 g de 1,3,3-triméthyl-2-[3-méthyl-1-(triméthylsilyloxy)-1,3-butadiényl]-1-cyclohexène (mélange isomères E/Z = 2,2:1 ; chromatographiquement pur à 61% ; 122 mmole) dans 400 ml de cyclohexane ont été pyrolysés comme il est décrit dans l'exemple 1. Le pyrolysat a été dissous dans le THF (250 ml) et traité avec HCl aqueux (50 ml $H_2O$ + 2,8 ml HCl concentré). Après 45 min, on a ajouté de l'eau et éther de pétrole. La phase organique a été lavée ($H_2O$, puis NaCl saturé), séchée sur $Na_2SO_4$, évaporée (33,5 g) et distillée (70°/6,7 Pa) pour fournir 30,6 g d'un mélange contenant 54% de la naphtalénone désirée, ainsi que du produit de départ. La distillation fractionnée de ce mélange a fourni des fractions contenant le cyclohexène de départ (9,0 g, p. eb. 53-60°/6,7 Pa, rend. estimé 27%) et des fractions riches en la naphtalénone susmentionnée (17,5 g, rend. 70%). La cristallisation (éther de pétrole/-30°) des fractions distillées les plus pures (14,2 g) a fourni cette naphtalénone à l'état cristallin (13,9 rend. 55%).

IR : 2920, 1665, 1465, 1435, 1375, 1360, 1225, 1155 $cm^{-1}$.

RMN($^1$H, 360MHz) : 1,00(s, 3H) ; 1,14(s, 3H) ; 1,15(s, 3H) ; 1,10-1,71(m, 6H) ; 1,87(s, 3H) ; 1,93(d, J = 18Hz, 1H) ; 2,07(s, 1H) ; 2,31(large d, J = 18Hz, 1H) ; 5,74(large s, 1H) δ ppm.

RMN($^{13}$C, 360MHz) : 18,3(t) ; 20,4(q) ; 21,3(q) ; 23,8(q) ; 32,1(s) ; 33,4(q) ; 38,6(s) ; 41,6(t) ; 43,8(t) ; 51,8(t) ; 61,6(d) ; 127,4(d) ; 155,7(s) ; 200,3(s) δ ppm.

SM : 206(8, $M^+$), 191(7), 163(5), 135(9), 123(100), 109(20), 82(23), 41(12).

Note odorante : décrite plus haut.

Le 1,3,3-triméthyl-2-[3-méthyl-1-(triméthylsilyloxy)-1,3-butadiényl]-1-cyclohexène de départ a été préparé comme suit :

a) à partir de β-cyclogéraniate de méthyle

Une solution de LDA (lithium diisopropylamine ; voir Exemple 1 a) ; 415 mmole) dans le THF/hexane (~ 650 ml) a été traitée avec chlorure de méthylallylmagnésium dans le THF (248 ml, 1,67N, 415 mmole). On a ajouté à la solution brun-gris ainsi obtenue du β-cyclogéraniate de méthyle (50,0 g, 275 mmole) dans le THF (50 ml) à 40°. Le mélange a été agité à 35-40° pendant 2 h et à 20° pendant 15 h. Le mélange de la réaction refroidi (-30°) a été traité avec $(CH_3)_3SiCl$ (88,5 g, 103,0 ml, 825 mmole). Après avoir retiré le bain de refroidissement, on a continué l'agitation pendant 2 h à 20°. Après le traitement décrit dans l'exemple 1 a) [27,8 g de $(C_2H_5)_3N$, 275 mmole], extraction et distillation (62-68°/6,7 Pa), on a obtenu 68,8 g d'un mélange (61% pur) d'isomères du cyclohexène désiré, dans les proportions E/Z = 2,2:1 (rend. 55%).

Données analytiques :

(E)-1,3,3-triméthyl-2-[3-méthyl-1-(triméthylsilyloxy)-1,3-butadiényl]-1-cyclohexène

RMN($^1$H, 360MHz) : 5,08(s, H vinyle) δ ppm.

(Z)-1,3,3-triméthyl-2-[3-méthyl-1-(triméthylsilyloxy)-1,3-butadiényl]-1-cyclohexène

RMN($^1$H, 360MHz) : 4,88(s, H vinyle) δ ppm.

NOE (⇝0,15) → pos. 1,71 ; 1,99 δ ppm.

NOE (⇝1,71) → pos. 0,15 ; 4,88 δ ppm.

NOE (⇝1,99) → pos. 0,15 ; 4,73 ; 4,88 δ ppm.

b) à partir de 3-méthyl-1-(2,2,5-triméthyl-1-cyclohexényl)-2-butén-1-one

On a procédé comme décrit dans l'exemple 1 b) en utilisant 1,0 g de buténone (4,85 mmole) dans 50 ml de THF et 5,40 ml (5,40 mmole) de $NaN[(CH_3)_3Si]_2$ dans 20 ml de THF, 0,68 ml (585 mg, 5,40 mmole) de $(CH_3)_3SiCl$. Après agitation pendant 1 h et le traitement décrit auparavant, suivi de distillation au four à boules (temp. bain 85°/8 Pa), on a obtenu 0,94 g (rend. 70%) de (Z)-1,3,3-triméthyl-2-[3-méthyl-1-(triméthylsilyloxy)-1,3-butadiényl]-1-cyclohexène dont les données analytiques étaient identiques à celles de l'isomère Z obtenu en a).

Exemple 4

Préparation de 4a,5,6,7,8,8a-hexahydro-3,4a,8,8-tétraméthyl-1(4H)-cis-naphtalénone

Ce composé a été obtenu par épimérisation de son isomère trans préparé dans l'Exemple 3 selon la méthode décrite dans l'Exemple 2. Après distillation on a obtenu 294 mg du produit désiré (rend. 59%).

IR($CDCl_3$) : 2940 (large), 1650, 1460, 1260, 1180 $cm^{-1}$.

RMN($^1H$, 360MHz, $CDCl_3$) : 0,83(s, 3H) ; 0,94(s, 3H); 0,99(s, 3H) ; 1,69(d, J = 20Hz, 1H) ; 1,75(s, 1H) ; 1,92(s, 3H) ; 2,68(d, J = 20Hz, 1H) ; 5,87(s, 1H) $\delta$ ppm.

RMN($^{13}C$, 360MHz, $CDCl_3$) : 16,6(t) ; 22,1(q) ; 24,2(q) ; 31,7(q) ; 32,8(q) ; 33,9(s) ; 34,9(s) ; 39,3(t) ; 40,7(t) ; 41,4(t) ; 62,1(d) ; 126,7(d) ; 158,9(s) ; 202,2(s) $\delta$ ppm.

SM : 206(7, $M^+$), 191(6), 135(8), 123(100), 109(15), 91(5), 82(18), 79(6), 67(6), 55(5), 41(7).

Note odorante : ambrée, sèche, puissante, légèrement naphtalénique.

Exemple 5

Préparation de perhydro-4a,8,8-triméthyl-1-trans-naphtalénone

Préparée à partir de la naphtalénone obtenue dans l'Exemple 1 (4,00 g, 20,8 mmole) dans l'éthanol (40 ml), par hydrogénation avec Pd/C (10%, 100 mg). Après 20 min (consommation $H_2$ : 480 ml), la suspension a été filtrée sur Celite®, concentrée et distillée au four à boules (85°/13,3 Pa). On a obtenu 3,90 g (rend. 97%) de perhydro-4a,8,8-triméthyl-1-trans-naphtalénone. Les données analytiques de ce produit coïncidaient avec celles publiées dans la littérature citée plus haut. Note odorante : décrite plus haut.

Exemple 6

Préparation de perhydro-3$\alpha$,4a$\alpha$,8,8-tétraméthyl-1-trans-naphtalénone

Obtenue par hydrogénation de la naphtalénone préparée dans l'Exemple 3 et suivant le procédé décrit dans l'Exemple 5.

P. eb. 90°/5,3 Pa.

IR : 2920, 1710, 1455, 1390, 1380 $cm^{-1}$.

RMN($^1H$, 360MHz) : 0,99(s, 3H) ; 1,01(s, 3H) ; 1,02(d, J = 7Hz, 3H) ; ~1,0-1,2(m, 1H) ; 1,20(s, 3H) ; 1,23-1,46(m, 4H) ; 1,50-1,67(m, 2H) ; 1,85(dd, J = 14, 7Hz, 1H) ; 1,98(dd, J = 16, 7Hz, 1H) ; 2,21(s, 1H) ; 2,33(o, J = 7Hz, 1H) ; 2,47(dd, J = 16, 6,5Hz, 1H) $\delta$ ppm.

RMN($^{13}C$, 360MHz) : 18,7(t) ; 21,5(q) ; 22,0(q) ; 25,4(q) ; 28,6(d) ; 32,2(s) ; 32,9(q) ; 37,9(s) ; 43,1(t) ; 43,3(t) ; 48,8(t) ; 50,5(t) ; 62,6(d) ; 212,6(s) $\delta$ ppm.

SM : 208(7, $M^+$), 193(6), 151(100), 125(23), 123(29), 109(13), 95(13), 81(19), 69(18), 67(16), 55(15), 41(21).

Note odorante : boisée, ambrée, faible.

Exemple 7

Préparation de perhydro-3$\beta$,4a$\alpha$,8,8-tétraméthyl-1-trans-naphtalénone

On a ajouté, à -5° et pendant 1 min, du $CH_3MgBr$ dans l'éther éthylique (3M, 2,2 ml, 6,6 mmole, Aldrich) à une suspension de CuI (50 mg) dans l'éther (10 ml). La solution verte a été agitée à -5° pendant 10 min. Une solution de la naphtalénone préparée dans l'Exemple 1 (1,16 g, 6,00 mmole) dans l'éther (10 ml) a été ajoutée goutte à goutte. Le mélange de la réaction a été agité pendant 90 min à -5°, versé sur un mélange de $NH_4Cl$ saturé/glace et extrait à l'éther de pétrole. La phase organique a été lavée ($H_2O$, puis NaCl saturé), séchée sur $Na_2SO_4$, concentrée (1,10 g) et distillée au four à boules (100°/10,6 Pa) pour fournir 850 mg de perhydro-3$\beta$,4a$\alpha$,8,8-tétraméthyl-1-trans-naphtalénone (96% pure, rend. 65%).

IR : 2920, 1705, 1455, 1385, 1360, 1225 $cm^{-1}$.

RMN($^1H$, 360MHz) : 0,92(s, 3H) ; 0,96(s, 3H) ; 0,97(d, J = 7Hz, 3H) ; ~1,0-1,2(m, 1H) ; 1,19(s, 3H) ; ~1,2-1,4(m, 3H) ; 1,38-1,47(m, 1H) ; 1,47-1,57(m, 2H) ; 1,57-1,72(m, 1H) ; 1,96-(large t, J = 12Hz, 1H) ; 2,04(s, 1H) ; 2,09(large m, 1H) ; 2,28(m, d, J = 12Hz, 1H) $\delta$ ppm.

| | |
|---|---|
| RMN($^{13}C$) : | 18,5(t) ; 21,2(q) ; 21,3(q) ; 22,5(q) ; 29,9(d) ; 32,0(s) ; 32,5(q) ; 39,6(s) ; 41,6(t) ; 43,3(t) ; 52,2(t) ; 53,7(t) ; 64,5(d) ; 211,7(s) δ ppm. |
| SM : | 208(25, $M^+$), 193(19), 165(12), 151(100), 137(10), 125(86), 109(19), 95(23), 81-(25), 69(30), 67(23), 55(23), 41(28). |

Note odorante : boisée-ambrée, faible, légèrement acide.

Exemple 8

Préparation de 4a,5,6,7,8,8a-hexahydro-4aα,8,8-triméthyl-1(2H)-trans-naphtalénone

Ce composé a été préparé par pyrolyse de 1,3,3-triméthyl-2-[1-(triméthylsilyloxy)-1,3-butadiényl]-1-cyclohexène (mélange E/Z = 3:1 ; 53 g) selon le procédé décrit dans l'Exemple 1. Une simple distillation (65°/9,3x10 Pa), suivie de distillation fractionnée et traitement avec quelques gouttes de HCl concentré a fourni, après chromatographie sur colonne de silice (20 g, cyclohexane/acétate d'éthyle 98:2), une fraction (147 mg) contenant 53% de la naphtalénone en titre.

On a également préparé le composé désiré à l'état pur à partir de la 4a,5,6,7,8,8a-hexahydro-4a,8,8-triméthyl-1(4H)-trans-naphtalénone obtenue dans l'Exemple 1, comme suit : une solution de cette naphtalénone (192 mg, 1,0 mmole) dans le THF (5 ml) a été ajoutée goutte à goutte à une solution refroidie (-30°) de $NaN[(CH_3)_3Si]_2$ (Aldrich, 1,0M dans le THF, 1,10 ml, 1,10 mmole) dans le THF (3 ml). Après 45 min, le mélange a été versé sur $H_2O$, puis extrait à l'éther/HCl aqueux à 5%. La phase organique a été lavée avec de l'eau, ensuite une solution aqueuse saturée de NaCl, séchée sur $Na_2SO_4$, concentrée et distillée au four à boules (70°/6,7 Pa). On a obtenu 155 mg (rend. 81%) de la naphtalénone désirée.

| | |
|---|---|
| IR : | 2920, 1715, 1650, 1460, 1390 $cm^{-1}$. |
| RMN($^1H$, 360MHz) : | 0,98(s, 3H) ; 1,01(s, 3H) ; 1,25(s, 3H) ; 1,08-1,19(m, 1H) ; 1,25(s, 3H) ; 1,33-1,72-(m, 5H) ; 2,40(s, 1H) ; 2,73(large d, J = 21Hz, 1H) ; 2,89(dt, J = 21, 3,5Hz, 1H) ; 5,52(dt, J = 8, 3,5Hz, 1H) ; 5,63(large d, J = 8Hz, 1H) δ ppm. |
| RMN($^{13}C$, 360MHz) : | 18,5(t) ; 21,4(q) ; 22,0(q) ; 32,1(s) ; 32,5(q) ; 39,4(t) ; 40,4(s) ; 42,5(t) ; 43,0(t) ; 63,8(d) ; 120,2(d) ; 141,8(d) ; 209,8(s) δ ppm. |
| SM : | 192(70, $M^+$), 177(58), 164(17), 149(27), 135(32), 121(14), 107(32), 93(30), 83-(38), 79(66), 67(20), 55(43), 41(100), 39(84). |

Exemple 9

Préparation de la 4a,7,8,8a-tétrahydro-4a,8,8-triméthyl-1(4H)-trans-naphtalénone

120,5 G de 2,6,6-trimethyl-1-[1-(triméthylsilyloxy)-1,3-butadiényl]-1,3-cyclohexadiène (460 mmole) dans le toluène (100 ml) ont été chauffés dans un autoclave à 220° (pression observée : 8 x$10^5$ Pa). Après 9 h, on a refroidi, concentré, mis en solution dans le THF (200 ml), traité avec HCl à 5% (30 ml) et agité pendant 1 h à 20°. Après distillation fractionnée (57°/6,7 Pa) et cristallisation (éther de pétrole/-78°), on a obtenu 12,1 g de la naphtalénone désirée (rend. 14%).

| | |
|---|---|
| IR (liq.) : | 2940, 1680, 1460, 1375, 1285, 1220 $cm^{-1}$. |
| RMN($^1H$, 360MHz) : | 1,10(s,3H) ; 1,15(s,3H) ; 1,20(s,3H) ; 1,75(dd,J = 17, 5Hz,1H) ; 1,95(d large, J = 17Hz,1H) ; 2,16(dd,J = 18, 6Hz,1H) ; 2,39(d large,J = 18Hz, 1H) ; 2,58(s,1H) ; 5,45(dd,J = 10, 2Hz,1H) ; 5,55(m,1H) ; 5,93(dd,J = 10, 3,5Hz, 1H) ; 6,65(m,1H) δ ppm. |
| RMN($^{13}C$) : | 20,3(q) ; 22,6(q) ; 30,3(s) ; 30,9(q) ; 39,9(s) ; 42,6(t) ; 44,5(t) ; 60,4(d) ; 123,4(d) ; 131,7(d) ; 134,8(d) ; 143,6(d) ; 200,8(s) δ ppm. |
| SM : | 190(27,$M^+$), 175(24), 157(10), 147(27), 134(28), 122(28), 107(100), 91(55), 83(47), 79(25), 77(25), 69(18), 65(17), 55(20), 41(20), 39(22). |

Note odorante : boisé-camphré, ambré, très fenchylique.

Le 2,6,6-triméthyl-1-[1-(triméthylsilyloxy)-1,3-butadiényl]-1,3-cyclohexadiène de départ a été préparé selon le procédé décrit dans l'Exemple 1b), à partir de la β-damascénone (98,9 g, 521 mmole). On a obtenu 120,5 g de produit (E/Z = 8:1; rend. 88%).

Isomère E

| | |
|---|---|
| RMN($^1H$, 360MHz) : | 0,24(s, 9H) ; 1,00(s,3H) ; 1,08(s,3H) ; 1,69(s,3H) ; 1,98(dd, J = 18, 2Hz,1H) ; 2,15-(d,J = 18Hz,1H) ; 4,76(dd,J = 10, 2Hz,1H) ; 4,99(dd, J = 17, 2Hz,1H) ; 5,54-(d,J = 10Hz,1H) ; 5,84(m,2H) ; 6,13(ddd,J = 17, 10, 10Hz,1H) δ ppm. |
| SM : | 262(9, $M^+$), 247(17), 219(15), 91(12), 75(19), 73(100), 45(15). |

Exemple 10

Préparation de 4a,5,6,7,8,8a-hexahydro-2,4a,8,8-tétraméthyl-1(4H)-trans-naphtalénone

8,00 G (28,8 mmole) de 1,3,3-triméthyl-2-[2-méthyl-(triméthylsilyloxy)-1,3-butadiényl]-1-cyclohexène dans le toluène (100 ml) ont été chauffés dans un autoclave à 220°. Après 9 h, on a refroidi, concentré, mis en solution dans le THF (20 ml), traité avec HCl concentré (0,5 ml) et agité pendant 1 h à 20°, Après distillation au four à boules (100°/13,3 Pa), le produit (4,30 g ; rend. 72%) a été repurifié par cristallisation à froid (éther de pétrole). On a obtenu 2,5 g de la naphtalénone désirée.

| | |
|---|---|
| IR (liq.) : | 2925, 1670, 1455, 1375, 1355 $cm^{-1}$. |
| RMN($^1$H, 360MHz) : | 1,00(s,3H) ; 1,14(s,3H) ; 1,17(s,3H) ; 1,19(m,1H) ; 1,39(m,3H) ; 1,59(m,2H) ; 1,74-(s large,3H) ; 2,04(dd,J=18, 6Hz,1H) ; 2,15(s,1H) ; 2,29(d large,J=18Hz,1H) ; 6,44(d large,J=6Hz,1H) δ ppm. |
| RMN($^{13}$C) : | 15,9(q) ; 16,3(t) ; 20,5(q) ; 21,4(q) ; 32,3(s) ; 33,4(q) ; 39,4(s) ; 41,9(t) ; 43,9(t) ; 46,4(t) ; 62,5(d) ; 136,1(s) ; 139,4(d) ; 200,9(s) δ ppm. |
| SM : | 206(17,$M^+$), 123(100), 109(18), 82(45), 41(12). |

Note odorante : ambrée-ambrinol, boisée, patchouli.

Le 1,3,3-triméthyl-2-[2-méthyl-1-(triméthylsilyloxy-1,3-butadiényl]-1-cyclohexène de départ a été préparé selon le procédé décrit dans l'Exemple 1b), à partir de (E)-2-méthyl-1(2,6,6-triméthyl-1-cyclohexén-1-yl)-2-butén-1-one (8,0 g ; 38,8 mmole ; peut être préparée selon le procédé décrit dans US 3,931,326). On a obtenu 8,30 g du produit désiré (E/Z = 9:1 ; rend. 77%).

| | |
|---|---|
| RMN($^1$H, 360MHz) : | 0,16(s,9H) ; 0,90(s,3H) ; 1,12(s,3H) ; 1,44(m,2H) ; 1,54(s,3H) ; 1,67(m,2H) ; 1,75-(s,3H) ; 1,98(m,2H) ; 4,78(d divisé,J=10Hz, 1H) ; 4,95(d divisé,J=18Hz,1H) ; 6,33(dd,J=18, 10Hz,1H) δ ppm. |

Exemple 11

Préparation de perhydro-2α,4aα,8,8-tétraméthyl-1-trans-naphtalénone

Ce composé a été préparé selon le procédé décrit dans l'Exemple 5, à partir de la naphtalénone obtenue dans l'Exemple 10 (1,0 g, 4,85 mmole). Après distillation au four à boules (100°/13,3 Pa), on a obtenu 1,00 g (rend. 99%) de la naphtalénone en titre.

| | |
|---|---|
| IR (liq.) : | 2920, 1705, 1450, 1380, 1355 $cm^{-1}$. |
| RMN($^1$H, 360MHz) : | 0,88(s,3H) ; 0,93(s,3H) ; 0,98(d,J=6,5Hz,3H) ; 1,10(dt, J=14, 3,5Hz,1H) ; 1,22-(s,3H) ; ~ 1,25-1,70(m,8H) ; 1,94(m,1H) ; 2,09(s,1H) ; 2,34(h,J=6,5Hz,1H) δ ppm. |
| RMN($^{13}$C) : | 14,6(q) ; 18,6(t) ; 20,6(q) ; 21,6(q) ; 32,3(t) ; 32,5(q) ; 41,2(2s) ; 41,7(t) ; 43,5(t) ; 44,5(t) ; 46,6(d) ; 65,3(d) ; 213,2(s) δ ppm. |
| SM : | 208(8,$M^+$), 193(6), 151(100), 123(18), 109(8), 95(10), 81(13), 67(12), 55(12), 41-(14). |

Note odorante : boisée, ambrée.

Exemple 12

Composition parfumante pour une eau de Cologne masculine

On a préparé une composition parfumante de base pour une eau de Cologne masculine en mélangeant les ingrédients suivants :

| Ingrédients | Parties en poids |
|---|---|
| Acétate de benzyle | 150 |
| Acétate de linalyle | 500 |
| Aldéhyde $C_{10}$ à 10% * | 150 |
| Essence de bergamote synthétique | 600 |
| Citral pur | 500 |
| Essence de citron | 1300 |
| Coumarine | 200 |
| Exaltex ® 1) | 500 |
| Essence de géranium Bourbon | 550 |
| Essence de lavandin | 1000 |
| Lilial ® 2) | 200 |
| Ethyl linalol | 500 |
| Musc ambrette | 500 |
| Oxyde de rose à 10% * | 500 |
| Essence de patchouli | 850 |
| Essence de petitgrain | 150 |
| Acétate de cédryle | 700 |
| Propionate de linalyle | 150 |
| Salicylate d'amyle | 100 |
| Salicylate de benzyle | 400 |
| Polysantol ® 3) à 10% * | 500 |
| Vertofix coeur 4) | 700 |
| Total | 10700 |

* dans le dipropylèneglycol (DIPG).
1) cyclopentadécanolide ; origine : Firmenich SA, Genève, Suisse.
2) 3-(4-tert-butyl-1-phényl)-2-méthylpropanal ; origine : L. Givaudan, Genève, Suisse.
3) (1'R,E)-3,3-diméthyl-5-(2',2',3'-triméthyl-3'-cyclopentén-1-yl)-4-pentén-2-ol ; origine : Firmenich SA, Genève, Suisse.
4) origine : International Flavors and Fragrances, USA.

L'adjonction, à cette composition de base, de 300 parties en poids de 4a,5,6,7,8,8a-hexahydro-4a,8,8-triméthyl-1(4H)-trans-naphtalénone donne à la composition nouvelle ainsi obtenue une note ambrée-boisée très caractéristique et exhalte le côté floral de la composition. L'adjonction, à la même composition de base, de 300 parties en poids de 4a,5,6,7,8,8a-hexahydro-3,4a,8,8-tétraméthyl-1(4H)-trans-naphtalénone confère à la nouvelle composition résultante une note moins masculine-boisée-ambrée que celle de la composition nouvelle précédente, mais lui permet de développer une note plus florale et plus douce.

Exemple 13

Composition parfumante pour détergent en poudre

On a préparé une composition parfumante de base pour un détergent en poudre par mélange des ingrédients suivants :

| Ingrédients | Parties en poids |
|---|---|
| Acétate de styrallyle | 1 |
| Acétate de carbinol | 3 |
| Aldéhyde hexylcinnamique | 20 |
| Ambrox ® 1) DL à 10% * | 1 |
| Acétate de verdyle | 3 |
| Acétate d'isononyle | 2 |
| Propionate de verdyle | 5 |
| Coumarine | 1 |
| Bourgeonal 2) à 50% * | 3 |
| Acétate de 4-tert-butyl-cyclohexyle 3) | 15 |
| Fleuramone ® 4) | 1 |
| Cyclométhylène citronellol | 5 |
| Isoraldéine ® 5) 70 P | 3 |
| Lilial ® 6) | 5 |
| Mayol ® 7) | 2 |
| Phénéthylol | 6 |

| | |
|---|---|
| Salicylate d'amyle | 3 |
| Tétrahydro muguol 8) | 3 |
| Dimyrcétol | 2 |
| α-Damascone à 10% * | 5 |
| Verdox ® 9) | 1 |
| Vertofix coeur 10) | 4 |
| Galaxolide ® 11) 50 | 5 |
| Total | 99 |

* dans le dipropylèneglycol (DIPG).

1) tétraméthyl perhydronaphtofuranne racémique ; origine : Firmenich SA, Genève, Suisse.
2) 3-(4-tert-butyl-phényl)-propanal ; origine : Naarden Int.
3) riche en isomère cis ; origine : Firmenich SA, Genève, Suisse.
4) 2-heptyl-1-cyclopentanone ; origine : International Flavors and Fragrances, USA.
5) iso-méthyl ionone ; origine : L. Givaudan, Genève, Suisse.
6) voir Exemple 12.
7) hydroxyméthyl isopropyl cyclohexane ; origine : Firmenich SA, Genève, Suisse.
8) mélange isomérique ; origine : International Flavors and Fragrances, USA.
9) 2-tert-butyl-1-cyclohexyl acétate ; origine : International Flavors and Fragrances, USA.
10) voir Exemple 12.
11) 1,3,4,6,7,8-hexahydro-4,6,6,7,8,8-hexaméthyl-cyclopenta[9]isochromène ; origine : International Flavors and Fragrances, USA.

L'adjonction, à cette composition de base, d'une partie en poids de 4a,5,6,7,8,8a-hexahydro-4a,8,8-triméthyl-1(4H)-trans-naphtalénone confère à la composition nouvelle ainsi obtenue une note boisée et terreuse. De plus, le côté floral se trouve nettement renforcé et il apparaît même un nouveau côté damasconé que l'on ne retrouve pas dans la composition de base.

Exemple 14

Composition parfumante pour un cosmétique

On a préparé une composition parfumante de base pour une préparation cosmétique par mélange des ingrédients suivants :

| Ingrédients | Parties en poids |
|---|---|
| Acétate de benzyle | 400 |
| Acétate de géranyle | 50 |
| Acétate de linalyle | 600 |
| Acétate de styrallyle | 50 |
| Aldéhyde $C_{11}$ à 10% * | 300 |
| Aldéhyde hexylcinnamique | 500 |
| Aldéhyde phénylacétique à 1% * | 200 |
| Essence de citron | 300 |
| Levocitrol | 200 |
| Coumarine | 100 |
| Cyclosal | 100 |
| Ethylvanilline à 1% * | 300 |
| Eugénol | 300 |
| Galaxolide ® 1) 50 | 1800 |
| Géraniol | 300 |
| Géranium synth. | 200 |
| Linalol | 600 |
| Iralia ® 2) | 100 |
| Hedione ® 3) | 300 |
| Essence de patchouli | 600 |
| Phénéthylol | 500 |
| Salicylate d'amyle | 400 |
| Salicylate de benzyle | 600 |
| Polysantol ® 4) à 10%* | 200 |
| α-Terpinéol nat. | 100 |
| Vertofix coeur 5) | 500 |
| Ylang synth. | 200 |
| Total | 9800 |

* dans le DIPG.
1) voir Exemple 13.
2) méthylionone (mélange d'isomères) ; origine : Firmenich SA, Genève, Suisse.
3) dihydrojasmonate de méthyle ; origine : Firmenich SA, Genève, Suisse.
4) voir Exemple 12.
5) voir Exemple 12.

Lorsqu'on a ajouté à cette composition de base de type boisé-ambré-musqué 200 parties en poids de perhydro-4a,8,8-triméthyl-1-trans-naphtalénone, on a obtenu une composition nouvelle dont le caractère boisé-patchouli chaud était nettement renforcé, le composé de l'invention lui ayant aussi conféré une nette connotation ambrée-musquée.

## Revendications

**1.** Cétone décalinique de formule

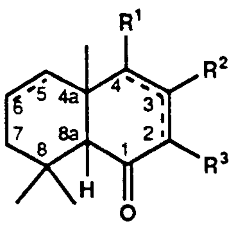

(I)

pouvant avoir une double liaison dans l'une des positions indiquées par les lignes pointillées ou deux doubles liaisons en positions 2 et 5 ou 3 et 5, telles qu'indiquées par les lignes pointillées, et dans laquelle les symboles $R^1$, $R^2$ et $R^3$ représentent un atome d'hydrogène ou un radical méthyle, à l'exclusion de la perhydro-4a,8,8-triméthyl-1-trans-naphtalénone.

**2.** Cétone décalinique selon la revendication 1, sous sa forme isomérique de formule

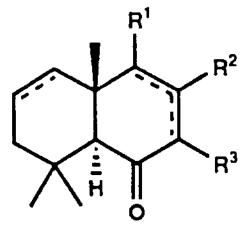

(Ia)

dans laquelle les lignes pointillées et les symboles $R^1$, $R^2$ et $R^3$ ont le sens indiqué à la formule (I), à l'exclusion de la perhydro-4a,8,8-triméthyl-1-trans-naphtalénone.

**3.** A titre de cétone décalinique selon la revendication 2, la 4a,5,6,7,8,8a-hexahydro-4a,8,8-triméthyl-1(4H)-trans-naphtalénone ou la 4a,5,6,7,8,8a-hexahydro-3,4a,8,8-tétraméthyl-1(4H)-trans-naphtalénone.

**4.** Utilisation d'une cétone décalinique de formule

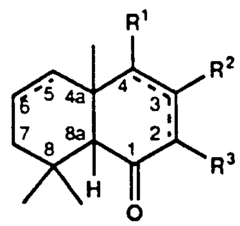

(I)

pouvant avoir une double liaison dans l'une des positions indiquées par les lignes pointillées ou deux doubles liaisons en positions 2 et 5 ou 3 et 5, telles qu'indiquées par les lignes pointillées, et dans laquelle les symboles $R^1$, $R^2$ et $R^3$ représentent un atome d'hydrogène ou un radical méthyle, à titre d'ingrédient parfumant.

**5.** Utilisation selon la revendication 4, caractérisée en ce que ladite cétone décalinique se présente sous une forme isomérique de formule

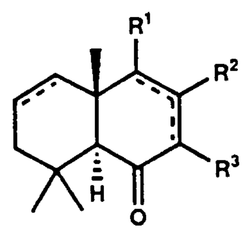

(Ia)

dans laquelle les lignes pointillées et les symboles $R^1$, $R^2$ et $R^3$ sont définis comme à la revendication 4.

**6.** Utilisation selon la revendication 5 de la perhydro-4a,8,8-triméthyl-1-trans-naphtalénone, de la 4a,5,6,7,8,8a-hexahydro-4a,8,8-triméthyl-1(4H)-trans-naphtalénone ou de la 4a,5,6,7,8,8a-hexahydro-3,4a,8,8-tétraméthyl-1(4H)-trans-naphtalénone.

**7.** Composition parfumante contenant, à titre d'ingrédient parfumant, une cétone décalinique de formule (I) ou (Ia) telle que définie à la revendication 4 ou, respectivement, 5.

**8.** Article parfumé contenant, à titre d'ingrédient parfumant, une cétone décalinique de formule (I) ou (Ia) telle que définie à la revendication 4 ou, respectivement, 5.

**9.** A titre d'article parfumé selon la revendication 8, un parfum ou une eau de toilette, un savon, un gel de douche ou bain, un shampoing, un déodorant corporel, une préparation cosmétique, un détergent ou un adoucissant textile ou un produit d'entretien.

**10.** Procédé pour la préparation d'une cétone décalinique de formule

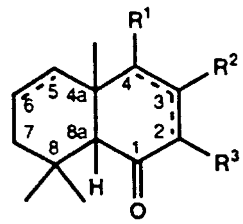

(I)

pouvant avoir une double liaison dans l'une des positions indiquées par les lignes pointillées ou deux doubles liaisons en positions 2 et 5 ou 3 et 5, telles qu'indiquées par les lignes pointillées, et dans laquelle les symboles $R^1$, $R^2$ et $R^3$ représentent un atome d'hydrogène ou un radical méthyle, caractérisé en ce qu'il comprend les étapes suivantes :

a) le traitement thermique d'un composé de formule

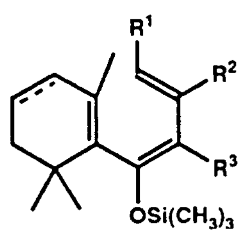

(II)

dans laquelle la ligne pointillée indique l'emplacement d'une liaison simple ou double et les symboles $R^1$, $R^2$ et $R^3$ sont définis comme à la formule (I), suivie d'un traitement acide du produit de réaction ainsi obtenu et de la séparation d'un composé de formule

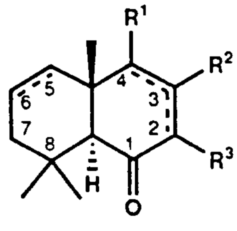

(Ia)

dans laquelle les symboles $R^1$, $R^2$ et $R^3$ ont le sens indiqué à la formule (I) et les lignes pointillées indiquent l'emplacement d'une liaison simple ou double en position 5 et d'une liaison double en position 2 ou 3 du cycle naphtalénique;

b) le cas échéant, l'épimérisation, suivant des procédés connus en soi, dudit composé de formule (Ia) définie en a) pour obtenir son isomère de configuration cis de formule

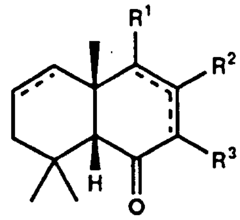

(Ib)

dans laquelle les lignes pointillées et les symboles $R^1$, $R^2$ et $R^3$ ont le sens indiqué à la formule (Ia) ; et

c) le cas échéant, la réduction sélective, de façon connue en soi, dudit composé de formule (Ia), respectivement de formule (Ib), pour obtenir un composé de formule

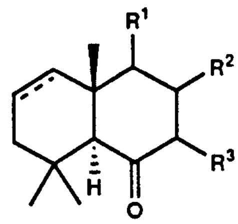

(I'a)

respectivement de formule

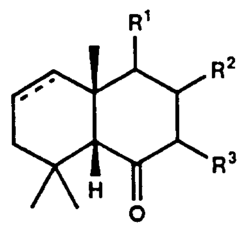

(I'b)

dans lesquelles la ligne pointillée indique l'emplacement d'une liaison simple ou double et les symboles $R^1$, $R^2$ et $R^3$ ont le sens indiqué ci-dessus.

**11.** Procédé selon la revendication 10, caractérisé en ce que le traitement thermique est une réaction de pyrolyse effectuée à une température comprise entre 300 et 400 °C.

**Claims**

**1.** Decalin ketone of formula

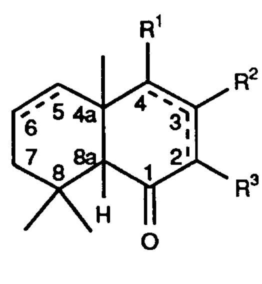

(I)

having a single or double bond in one of the positions indicated by the dotted lines, or two double

bonds in positions 2 and 5 or 3 and 5 such as indicated by the dotted lines, and wherein symbols $R^1$, $R^2$ and $R^3$ represent a hydrogen atom or a methyl radical, excluding perhydro-4a,8,8-trimethyl-1-trans-naphthalenone.

**2.** Decalin ketone according to claim 1, in its isomeric form of formula

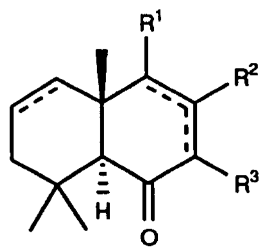

(Ia)

wherein the dotted lines and symbols $R^1$, $R^2$ and $R^3$ have the meaning indicated in formula (I), excluding perhydro-4a,8,8-trimethyl-1-trans-naphthalenone.

**3.** As a decaline ketone according to claim 2, 4a,5,6,7,8,8a-hexahydro-4a,8,8-trimethyl-1(4H)-trans-naphthalenone or 4a,5,6,7,8,8a-hexahydro-3,4a,8,8-tetramethyl-1(4H)-trans-naphthalenone.

**4.** Use of a decalin ketone of formula

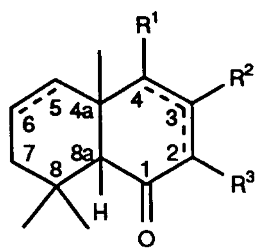

(I)

having a single or double bond in one of the positions indicated by the dotted lines, or two double bonds in positions 2 and 5 or 3 and 5 such as indicated by the dotted lines, and wherein symbols $R^1$, $R^2$ and $R^3$ represent a hydrogen atom or a methyl radical, as a perfuming ingredient.

**5.** Use according to claim 4, characterized in that said decalin ketone possesses an isomeric form of formula

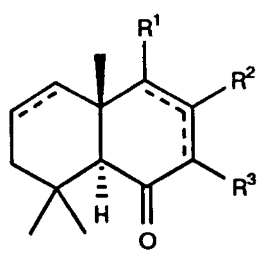

(Ia)

wherein the dotted lines and symbols $R^1$, $R^2$ and $R^3$ are defined as in claim 4.

**6.** Use according to claim 5 of perhydro-4a,8,8-trimethyl-1-trans-naphthalenone, 4a,5,6,7,8,8a-hexahydro-4a,8,8-trimethyl-1(4H)-trans-naphthalenone or 4a,5,6,7,8,8a-hexahydro-3,4a,8,8-tetramethyl-1(4H)-trans-naphthalenone.

7. Perfuming composition containing as a perfuming ingredient a decalin ketone of formula (I) or (Ia) such as defined in claim 4 or, respectively 5.

8. Perfumed article containing as a perfuming ingredient a decalin ketone of formula (I) or (Ia) such as defined in claim 4, respectively 5.

9. As a perfumed article according to claim 8, a perfume or a Cologne, a soap, a shower or bath gel, a shampoo, a body deodorant, a cosmetic preparation, a detergent or a fabric softener or a household product.

10. Process for the preparation of a decalin ketone of formula

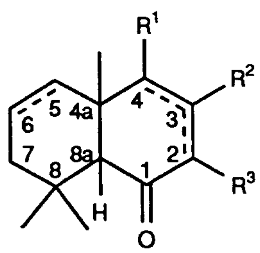

(I)

having a single or double bond in one of the positions indicated by the dotted lines, or two double bonds in positions 2 and 5 or 3 and 5 such as indicated by the dotted lines, and wherein symbols $R^1$, $R^2$ and $R^3$ represent a hydrogen atom or a methyl radical, characterized in that it comprises the following steps:

a) the thermal treatment of a compound of formula

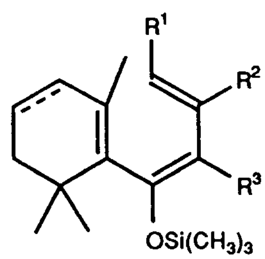

(II)

wherein the dotted line indicates the location of a single or double bond and symbols $R^1$, $R^2$ and $R^3$ are defined as in formula (I), followed by an acidic treatment of the reaction product thus obtained and the separation of a compound of formula

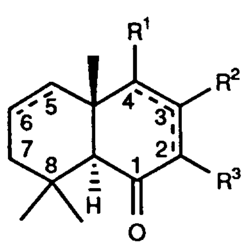

(Ia)

wherein symbols $R^1$, $R^2$ and $R^3$ have the meaning indicated in formula (I) and the dotted lines indicate the location of a single or double bond in position 5 and of a double bond in position 2 or 3 of the naphthalene ring ;

b) if necessary, the epimerisation, according to generally known methods, of said compound of formula (Ia) defined in a) to yield its cis-configuration isomer of formula

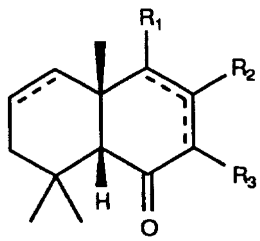

(Ib)

wherein the dotted lines and symbols $R^1$, $R^2$ and $R^3$ have the meaning indicated in formula (Ia) ; and

c) if necessary, the selective reduction, in a generally known manner, of said compound of formula (Ia), respectively of formula (Ib), to obtain a compound of formula

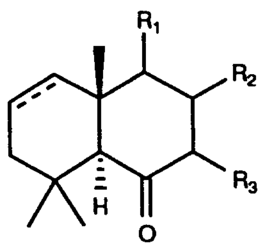

(I'a)

respectively of formula

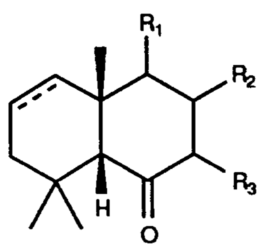

(I'b)

wherein the dotted line indicates the location of a single or double bond and symbols $R^1$, $R^2$ and $R^3$ have the meaning indicated above.

**11.** Process according to claim 10, characterized in that said thermal treatment is a pyrolysis reaction carried out at a temperature of between 300 and 400 °C.

**Patentansprüche**

1. Ketodekalin der Formel

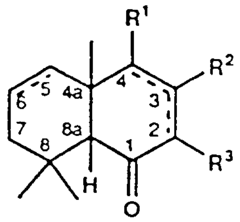

(I)

das eine Doppelbindung in einer der durch die punktierten Linien angezeigten Stellungen oder zwei Doppelbindungen in den Stellungen 2 und 5 oder 3 und 5 besitzen kann, wie dies durch die punktierten Linien dargestellt wird, und worin die Symbole $R^1$, $R^2$ und $R^3$ ein Wasserstoffatom oder einen Methylrest bedeuten, mit Ausnahme des Perhydro-4a,8,8-trimethyl-1-trans-naphthalinons.

2. Ketodekalin gemäss Patentanspruch 1, in Form seines Isomeren der Formel

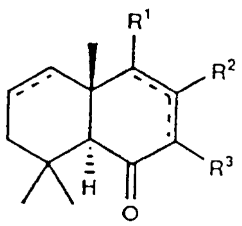

(Ia)

worin die punktierten Linien und die Symbole $R^1$, $R^2$ und $R^3$ die in der Formel (I) angegebene Bedeutung besitzen, mit Ausnahme des Perhydro-4a,8,8-trimethyl-1-trans-naphthalinons.

3. Als Ketodekalin gemäss Patentanspruch 2, das 4a,5,6,7,8,8a-Hexahydro-4a,8,8-trimethyl-1(4H)-trans-naphthalinon oder das 4a,5,6,7,8,8a-Hexahydro-3,4a,8,8-tetramethyl-1(4H)-trans-naphthalinon.

4. Verwendung eines Ketodekalins der Formel

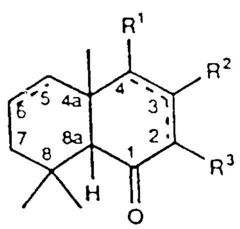

(I)

das eine Doppelbindung in einer der durch die punktierten Linien angegebenen Stellung oder zwei Doppelbindungen in den Stellungen 2 und 5 oder 3 und 5 besitzen kann, wie dies durch die punktierten Linien dargestellt wird, und worin die Symbole $R^1$, $R^2$ und $R^3$ ein Wasserstoffatom oder einen Methylrest bedeuten, als Riechstoffbestandteil.

5. Verwendung gemäss Patentanspruch 4, dadurch gekennzeichnet, dass das genannte Ketodekalin die Form des Isomeren der Formel

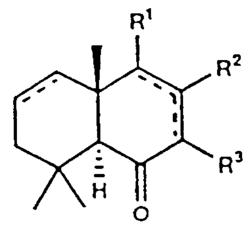

(Ia)

aufweist, worin die punktierten Linien und die Symbole $R^1$,$R^2$ und $R^3$ wie im Anspruch 4 definiert sind.

6. Verwendung gemäss Patentanspruch 5 des Perhydro-4a,8,8-trimethyl-1-trans-naphthalinons, des 4a,5,6,7,8,8a-Hexahydro-4a, 8,8-trimethyl-1(4H)-trans-naphthalinons oder des 4a,5,6,7,8,8a-Hexahydro-3,4a,8,8-tetramethyl-1(4H)-trans-naphthalinons.

7. Riechstoffkomposition, enthaltend als Riechstoffbestandteil ein Ketodekalin der Formel (I) oder (Ia), wie sie in den Patentansprüchen 4 beziehungsweise 5 definiert sind.

8. Parfümierter Artikel, enthaltend als Riechstoffbestandteil ein Ketodekalin der Formel (I) oder (Ia), wie sie in den Patentansprüchen 4 beziehungsweise 5 definiert sind.

9. Als parfümierter Artikel gemäss Patentanspruch 8, ein Parfüm oder ein Toilettwasser, eine Seife, ein Dusch- oder Badegel, ein Shampoo, ein Körperdesodorant, eine kosmetische Zusammensetzung, ein Detergent oder ein Textilauffrischungsmittel oder ein Pflegeprodukt.

10. Verfahren zur Herstellung eines Ketodekalins der Formel

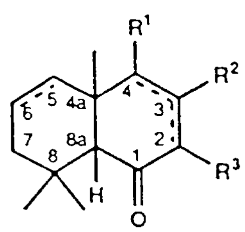

(I)

das eine Doppelbindung in einer durch die punktierten Linien angezeigten Stellung oder zwei Doppelbindungen in den Stellungen 2 und 5 oder 3 und 5 besitzen kann, wie dies durch die punktierten Linien dargestellt wird, und worin die Symbole $R^1$, $R^2$ und $R^3$ ein Wasserstoffatom oder einen Methylrest bedeuten, dadurch gekennzeichnet, dass es die folgenden Stufen umfasst:

a) die thermische Behandlung einer Verbindung der Formel

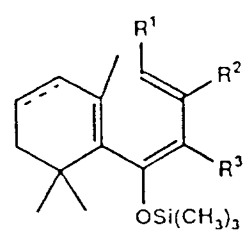

(II)

worin die punktierte Linie die Stelle einer Einfachbindung oder einer Doppelbindung anzeigt und die Symbole $R^1$, $R^2$ und $R^3$ wie in der Formel (I) definiert sind , gefolgt von einer Säurebehandlung des so erhaltenen Reaktionsproduktes und der Abtrennung einer Verbindung der Formel

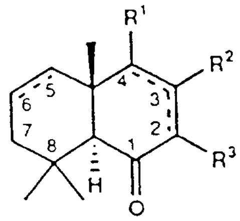

(Ia)

worin die Symbole $R^1$, $R^2$ und $R^3$ die in der Formel (I) angegebene Bedeutung besitzen und die punktierten Linien die Stelle einer Einfachbindung oder einer Doppelbindung in Stellung 5 und einer Doppelbindung in den Stellungen 2 oder 3 des Naphthalinringes anzeigen;

b) gegebenenfalls die gemäss an sich bekannter Verfahren durchgeführte Epimerisierung der genannten, in a) definierten Verbindung der Formel (Ia), um ihr Cis-Isomeres der Formel

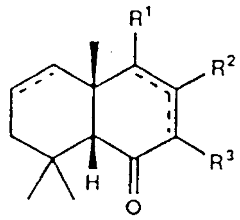

(Ib)

zu erhalten, worin die punktierten Linien und die Symbole $R^1$, $R^2$ und $R^3$ die in der Formel (Ia) angegebene Bedeutung besitzen;

und

c) gegebenenfalls die auf an sich bekannter Weise durchgeführte selektive Reduktion der genannten Verbindung der Formel (Ia), beziehungsweise der Formel (Ib), um eine Verbindung der Formel

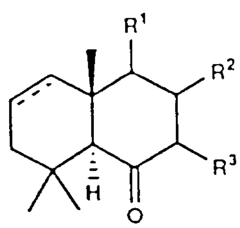

(I'a)

beziehungsweise der Formel

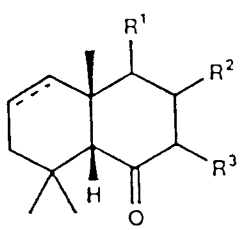

(I'b)

zu erhalten, worin die punktierte Linie die Stelle einer Einfachbindung oder einer Doppelbindung anzeigt, und die Symbole $R^1$, $R^2$ und $R^3$ die oben angegebene Bedeutung besitzen.

**11.** Verfahren gemäss Patentanspruch 10, dadurch gekennzeichnet, dass die thermische Behandlung eine Pyrolysereaktion ist, die bei einer Temperatur, die Temperaturen zwischen 300 und 400 C umfasst, durchgeführt wird.